# EUROPÄISCHE PATENTSCHRIFT

(11) **EP 0 599 307 B1**
(45) Veröffentlichungstag und Bekanntmachung des Hinweises auf die Patenterteilung: **26.08.1998**
(21) Anmeldenummer: 93118997.1
(22) Anmeldetag: 25.11.1993
(51) Int. Cl.: A61K 35/78

(54) **Johanniskraut-Trockenextrakt, Verfahren zu seiner Herstellung und seine Verwendung**
St.-John's-wort dry extract, process for its preparation and its use
Extrait sec de l'herbe de la Saint Jean, procédé de sa préparation et son utilisation

(30) Priorität: 27.11.1992 DE 4239959
(43) Veröffentlichungstag der Anmeldung: 01.06.1994
(73) Patentinhaber: Dr. Willmar Schwabe GmbH & Co., D-76227 Karlsruhe (DE)
(72) Erfinder: Chatterjee, Shyam Sunder, Dr., D-76139 Karlsruhe (DE); Erdelmeier, Clemens, Dr., D-76139 Karlsruhe (DE); Stumpf, Heinz, Dr., D-76228 Karlsruhe (DE)
(74) Vertreter: Bunke, Holger, Dr.rer.nat. Dipl.-Chem.

(56) Entgegenhaltungen:
- DE-A- 1 569 849
- DE-A- 3 935 772
- CHEMICAL ABSTRACTS, Band 107, Nr. 22, 30. November 1987, Columbus, Ohio, USA STOYANOVA, A. et al. "Thin- -layer chromatography of extracts of Hypericum perfor- atum"
- CHEMICAL ABSTRACTS, Band 105, Nr. 4, 28. Juli 1986, Columbus, Ohio, USA GEORGIEV, E. et al.: "Effect of solvent and moisture of St.-John's-wort on extraction of some biologically active substances. I. Extraction of hypericin with glycerides and ethyl alcohol. "
- CHEMICAL ABSTRACTS, Band 105, Nr. 4, 28. Juli 1986, Columbus, Ohio, USA GEORGIEV, E. et al.: " Effect of solvent and moisture of St.-John's-wort on extraction of some biologically active substances. II. Extraction of hypericin with glycol. "
- CHEMICAL ABSTRACTS, Band 101, Nr. 22, 26. November 1984, Columbus, Ohio, USA GEORGIEV, E. et al.: "Extrac- tion of Hypericum perforatum L. "

## Beschreibung

Die Erfindung betrifft einen Trockenextrakt aus dem Kraut von Hypericum perforatum L. (Johanniskraut), in dem die stark photosensibilisierenden Verbindungen des Naphthodianthron-Typs selektiv abgereichert oder ganz eliminiert wurden. Die Erfindung betrifft ferner Verfahren zur Herstellung des Trockenextrakts und seine Verwendung als Arzneimittel.

Johanniskraut wird schon lange als Arzneidroge verwendet. Seit neuerem wird die Droge auch als Sedativum und Anxiolytikum sowie zur Behandlung depressiver Verstimmungen verwendet. Therapeutisch eingesetzt werden in der Regel wäßrig-ethanolische Extrakte aus Johanniskraut und ölige Zubereitungen.

Bisher wurden aus Johanniskraut folgende Inhaltsstoffe bzw. Inhaltsstoffgruppen isoliert: Ätherische Öle (0,1 % - 0,3 %), Catechin-Gerbstoffe (ca. 10 %), Flavone und Xanthone (2 % - 4 %), Naphthodianthrone (Hypericine) (0,1 %) sowie Pflanzensäuren. Darüber hinaus enthalten die Frischpflanze und schnell und schonend getrocknetes Johanniskraut Hyperforin (1,0 - 1,4 %) und in geringerem Maße Adhyperforin. Hyperforin besitzt ausgeprägte antibiotische Eigenschaften und scheint an der sedierenden Wirkung des Johanniskrauts beteiligt zu sein (vgl. P. Maisenbacher, "Untersuchungen zur Analytik von Johanniskrautöl", Dissertation Universität Tübingen 1991). Das Vorkommen von Anthrachinonen im Johanniskraut ist dagegen nicht bewiesen.

Hypericin, Hyperforin und Adhyperforin besitzen die folgenden Strukturformeln:

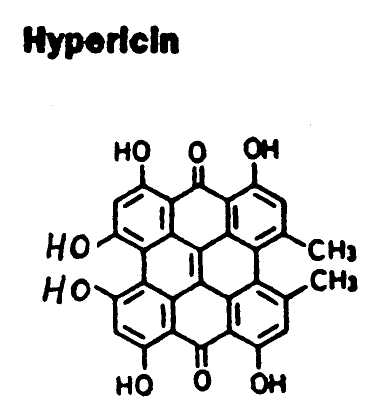

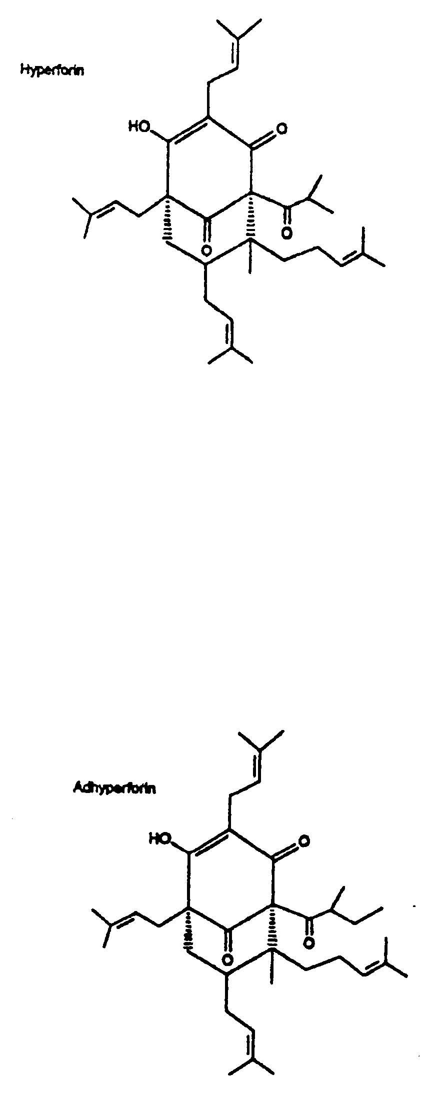

Das Bundesgesundheitsamt führt eine positive Monographie "Hyperici herba", die neben der Erwähnung der sedativen, anxiolytischen und antidepressiven Indikationen auch ausdrücklich auf die Nebenwirkung der Photosensibilisierung durch Johanniskraut hinweist. Diese ist zwar bisher hauptsächlich für Weidetiere (z.B. Rinder, Schafe, Pferde) beschrieben, wobei die Nebenwirkung nicht selten tödlich verlaufen ist. Einzelne Fälle, allerdings ohne tödlichen Ausgang, wurden auch beim Menschen beschrieben (Hausen, B., Allergiepflanzen - Pflanzenallergene, Handbuch und Atlas der allergieinduzierenden Wild- und Kulturpflanzen, ecomed, Landsberg-München 1988).

Jahrzehntelang wurden Hypericin und seine Derivate als Hauptwirkstoff von Hypericum perforatum angesehen. Es wurden daher viele Anstrengungen unternommen, reines Hypericin aus Hypericum perforatum herzustellen (C. Czerny, Hoppe-Seyler's Z. physiol. Chem. 73, 371 (1911)); (H. Brockmann, M.N. Haschad, K. Maier und F. Pohl, Naturwissenschaften 27, 550 (1939)); (N. Pace und G. Machinney, J. Am. Chem. Soc. 63, 2570 (1941)). Der Arbeitsgruppe von Brockmann gelang 1950 / 51 der endgültige Strukturbeweis und die Totalsynthese von Hypericin.

Die Herstellung angereicherter Extrakte wurde ebenfalls mit der Zielsetzung betrieben, eine Anreicherung des Hypericins zu erreichen (DE-PS 1 569 849). Niesel und Schilcher beschreiben Methoden zur Anreicherung von Extrakten aus Hypericum perforatum mit dem Ziel, zu einem hohen Anteil von Hypericin zu kommen (Arch. Pharm. 323, 755 (1990)).

In Übereinstimmung mit diesen wissenschaftlichen Ergebnissen hat die Kommission E des Bundesgesundheitsamtes eine Monographie erstellt (Bundesanzeiger Nr. 228 vom 05.12.84), in der die Menge an Gesamt-Hypericin (Hypericin und Pseudohypericin), nämlich 0,2 mg bis 1 mg, als Basis für die Ermittlung der Tagesdosis bei Zubereitungen aus Hypericum gewählt wird. Dies weicht von vergleichbaren Monographien ab, bei denen in der Regel allein die Menge an Droge Basis für die einzusetzende Menge an Zubereitung ist. Dies zeigt, daß dem Inhaltsstoff Hypericin bei den Anwendungsgebieten psychovegetative Störungen, depressive Verstimmungszustände, Angst und nervöse Unruhe die alleinige Ursache für die Wirksamkeit hypericumhaltiger Zubereitungen zugemessen wurde. Dies wird auch noch in der neueren Literatur bestätigt (R. Hänsel, Phytopharmaka, Springer Verlag S. 260); (S. Niesel, H. Schilcher Arch. Pharm. 323, 755 (1990)); (Literatursammlung Hypericum perforatum, M. Wichtl, 1983, S. 8).

Der Erfindung liegt die Aufgabe zugrunde, einen Johanniskraut-Trockenextrakt bereitzustellen, der möglichst wenig Hypericin oder ähnliche photosensibilisierende Verbindungen enthält, aber dennoch die bisher dem Hypericin zugeschriebene Wirksamkeit besitzt, sowie Verfahren zu seiner Herstellung, die möglichst einfach und ohne großen apparativen Aufwand durchführbar sein sollen und mit denen es gelingt, Hypericine sehr stark abzureichern, aber erwünschte Komponenten wie Hyperforin und Adhyperforin, die kein photosensibilisierendes Potential aufweisen, in demselben Extrakt anzureichern.

Diese Aufgabe wird erfindungsgemäß durch den Trockenextrakt gemäß einem der Patentansprüche 1 - 4, die Verfahren gemäß den Patentansprüchen 8 - 16 sowie durch die Verwendung des Extrakts als Arzneimittel gelöst.

Der Erfindung liegt die überraschende Erkenntnis zugrunde, daß sich ein hypericinfreier oder hypericinarmer, gleichzeitig an Gesamt-Hyperforin (Hyperforin und Adhyperforin) angereicherter Extrakt aus Hypericum perforatum sowohl aus einer alkoholischen Primärextraktlösung durch einfaches Ausrühren mit Polyvinylpyrrolidon oder Aktivkohle als auch säulenchromatographisch ohne besonderen Trennaufwand herstellen läßt, wenn ein Dioxan-Wasser-Gemisch als Eluens verwendet wird.

Die erfindungsgemäß hergestellten Extrakte und die daraus gewonnenen pharmazeutischen Zubereitungen haben den großen Vorteil, frei von den für Hypericin beschriebenen Nebenwirkungen zu sein, gleichzeitig aber die bisher offenbar irrtümlich dem Hypericin zugeschriebene volle Wirksamkeit als Psychovegetativa und Antidepressiva zu besitzen.

Während handelsübliche Präparate Extrakte mit einem Gehalt von 0,12 % - 0,64 % Gesamthypericin (Angaben entsprechend Roter Liste 1992) enthalten, sind bevorzugte Ausführungsformen des erfindungsgemäßen Trockenextraktes durch einen Gesamthypericingehalt von weniger als 0,1 Gew.-%, besonders bevorzugt von weniger als 0,03 Gew.-% oder von Null, sowie durch einen erhöhten Gesamt-Hyperforingehalt, welcher je nach Ausgangsdroge mindestens 5, vorzugsweise mindestens 10 Gew.-% beträgt, gekennzeichnet.

Der erfindungsgemäße Trockenextrakt wird vorzugsweise zur Herstellung psychovegetativ und antidepressiv wirksamer Arzneimittel verwendet, die sowohl in der Human- als auch in der Veterinärmedizin eingesetzt werden können. Gegenstand der Erfindung sind somit auch psychovegetativ und antidepressiv wirksame Arzneimittel, die einen erfindungsgemäßen Trockenextrakt enthalten, gegebenenfalls zusammen mit üblichen, pharmakologisch verträglichen Hilfs- und Zusatzstoffen. Diese Arzneimittel können in Form von Tabletten, Dragees, Hart- und Weichgelatine-Kapseln, in gelöster Form als Tropfen etc. vorliegen.

Überraschenderweise wurde festgestellt, daß erfindungsgemäße Extrakte, in denen Hyperforin und Adhyperforin besonders hoch angereichert waren, eine stark serotonin-antagonistische Wirksamkeit aufweisen. Erfindungsgemäße Extrakte mit hohem Gesamt-Hyperforingehalt werden deshalb mit besonderem Vorteil als Arzneimittel mit serotonin-antagonistischer Wirksamkeit verwendet, gegebenenfalls zusammen mit üblichen, pharmakologisch verträglichen Hilfs- und Zusatzstoffen.

Bei einer bevorzugten Ausführungsform des erfindungsgemäßen Verfahrens zur Herstellung des Trockenextraktes wird ein alkoholischer oder wäßrig-alkoholischer Gesamtextrakt (Primärextrakt) von einer frischen oder schonend getrockneten Johanniskraut-Droge direkt nach dem Abfiltrieren des Drogenrückstands ohne Zwischentrocknung mit Polyvinylpyrrolidon (PVP) oder Aktivkohle versetzt, ausgerührt und anschließend durch Filtration von PVP oder Aktivkohle abgetrennt und getrocknet. Es hat sich dabei überraschenderweise gezeigt, daß die Hypericine sehr stark an PVP oder Aktivkohle adsorbiert werden. Vorzugsweise setzt man dem flüssigen Primärextrakt 30 bis 200 % PVP oder 5 bis 50 % Aktivkohle zu, jeweils bezogen auf die Masse des Trockenextrakts.

Bei einer anderen bevorzugten Ausführungsform des erfindungsgemäßen Verfahrens zur Herstellung des Trockenextrakts wird ein wäßrig-ethanolischer Gesamtextrakt (Primärextrakt) von Johanniskraut-Droge in einem Dioxan-Wasser-Gemisch, vorzugsweise im Volumenverhältnis 7 : 3, gelöst und über handelsüblichen Polysaccharid als Trägermaterial, z.B. über Sephadex -LH-20, gelchromatographisch fraktioniert. Es hat sich überraschenderweise gezeigt, daß die Hypericine bei Verwendung von Dioxan-Wasser als Eluens, vorzugsweise ebenfalls im Volumenverhältnis 7 : 3, als letzte Substanzen von der Säule eluieren. Das Eluat wird unfraktioniert aufgefangen bis zur beginnenden Elution der violett bis blutrote Zonen. Diese Naphthodianthron-Fraktion wird getrennt aufgefangen und verworfen, während das zuvor aufgefangene Eluat zur Trockne eingeengt und gegebenenfalls nachgetrocknet wird.

Bei einer weiteren bevorzugten Ausführungsform des erfindungsgemäßen Verfahrens wird ein wäßrig-ethanolischer Gesamtextrakt von Johanniskraut zunächst zwischen n-Butanol und Wasser verteilt. Die n-Butanolphase wird zur Trockne eingeengt und enthält 35 % - 40 % des eingesetzten Gesamtextraktes. Die Wasserphase enthält 60 % - 65 % des eingesetzten Gesamtextraktes mit folgenden Gehalten:
- 0,5 % - 1 %: Hyperosid
- < 0,01 %: Quercetin
- < 0,01 %: I3,II8 Biapigenin
- < 0,01 %: Amentoflavon
- < 0,02 %: Hypericin
- < 0,02 %: Pseudohypericin

Die getrocknete n-Butanolphase wird dann gelchromatographisch weiterfraktioniert, wie oben beschrieben.

Bei allen erfindinngsgemäßen Verfahrensvarianten wird der Primärextrakt vorzugsweise durch Extraktion der Droge mit 50 bis 96 %igem (Gew.-%) Ethanol gewonnen.

### Beispiel 1

1 kg schonend getrocknetes und grob gepulvertes Johanniskraut werden mit 7 kg 96 %igem Ethanol versetzt und eine Stunde bei 55°C intensiv gerührt. Anschließend wird über ein Filter abgesaugt und der Drogenrückstand in der gleichen Weise noch ein zweites Mal extrahiert. Die beiden Extraktlösungen werden vereinigt, und mit einem aliquot wird der Trockenrückstand bestimmt. Es ergibt sich ein Trockenrückstand von 23 %, entsprechend einer Ausbeute von 231 g Trockenextrakt aus 1 kg Droge. Der Gesamt-Hypericingehalt in diesem Extrakt beträgt 0,41 %, der Hyperforingehalt 6,3 %. Es werden der Extraktlösung dann 115 g Polyvinylpyrrolidon (PVP : Trockenextrakt = 1:2) zugegeben, und bei Raumtemperatur wird eine Stunde lang gerührt. Danach wird über ein Filter abgesaugt und die erhaltene Extraktlösung bei 55°C unter vermindertem Druck zur Trockne eingeengt. Es wird anschließend bei 45°C im Vakuum nachgetrocknet. Es resultieren 189 g Trockenextrakt. In diesem Extrakt liegt der Gesamt-Hypericingehalt unter 0,015 %, der Gesamt-Hyperforingehalt bei 8,7 %.

### Beispiel 2

1 kg schonend getrocknetes und grob gepulvertes Johanniskraut werden mit 7 kg 60 %igem Ethanol versetzt und 1 Stunde bei 55°C intensiv gerührt. Anschließend wird über ein Filter abgesaugt und der Drogenrückstand in der gleichen Weise noch ein zweites Mal extrahiert. Die beiden Extraktlösungen werden vereinigt, und mit einem aliquot wird der Trockenrückstand bestimmt. Es ergibt sich ein Trockenrückstand von 35 %, entsprechend einer Ausbeute von 355 g Trockenextrakt aus 1 kg Droge, mit einem Gesamthypericingehalt von 0,13 %. Der Extraktlösung werden 35 g Aktivkohle zugegeben, und während einer Stunde wird bei Raumtemperatur gerührt. Es wird wiederum filtriert und die Lösung in der gleichen Weise, wie in Beispiel 1 beschrieben, zur Trockne gebracht. Es ergeben sich 337 g Trockenextrakt mit einem Gesamthypericingehalt unter 0,015 %.

### Beispiel 3

2 kg schonend getrocknetes und gepulvertes Johanniskraut werden mit 20 kg 60 %igem (Gew.-%) Ethanol versetzt und eine Stunde bei 60°C intensiv gerührt. Anschließend wird über einen Filter abgesaugt und der Drogenrückstand nochmals in der gleichen Weise behandelt. Die beiden Extrakte werden vereinigt und unter vermindertem Druck bei 60°C zur Trockne eingeengt. Anschließend wird bei 45°C nachgetrocknet. Es resultieren 732,4 g Trockenextrakt.

Ein Gewichtsteil dieses Trockenextraktes wird in vier Volumenteilen Dioxan-Wasser 7:3 (Vol./Vol.) unter leichter Erwärmung gelöst. Diese Lösung wird auf eine mit Sephadex LH-20 beschickte und mit Dioxan-Wasser 7:3 (Vol./Vol.) Chromatographiesäule aufgetragen. Das Verhältnis von Ausgangsextrakt zu Trägermaterial beträgt dabei 1:13. Das Elutionsmittel ist Dioxan-Wasser 7:3 (Vol./Vol.). Es wird eluiert und unfraktioniert aufgegangen, bis Zonen von der Säule eluieren, die sich nach Auftrennung im Dünnschichtchromatogramm mit Flavognost-Reagens am Tageslicht blau oder grün färben (Hypericin-Derivate). Letzterwähnte Zonen werden getrennt aufgegangen. Es werden so zwei Fraktionen erhalten: Fraktion 1, 97,2 % des eingesetzten Extraktes, und Fraktion 2, 0,5 % des eingesetzten Extraktes. Während Fraktion 2, die die Hypericin-Derivate enthält, verworfen wird, wird die hypericinarme oder auch hypericinfreie Fraktion 1 zur Trockne eingeengt und gegebenenfalls nachgetrocknet. Die so erhaltene Trockensubstanz stellt den erfindungsgemäßen Trockcnextrakt mit vermindertem Gesamt-Hypericingehalt dar.

Die Wirkung eines handelsüblichen Extraktes mit einem Gesamt-Hypericin-Gehalt von 0,17 % (Extrakt A) wurde mit dem gemäß Beispiel 1 hergestellten, hypericinarmen Extrakt (Extrakt B) verglichen. Dieser enthält Gesamt-Hypericin in einer Menge, die bei der analytischen Nachweisgrenze lag, nämlich unter 0,015 Gew.-%.

Zum Nachweis der Wirksamkeit des erfindungsgemäß hergestellten Extrakts wurde dessen Serotonin-Rezeptor-antagonistische Wirkung geprüft, und zwar am Modell des 5-HT₃-Rezeptors.

Das Vorhandensein und die physiologische sowie die pathologische Bedeutung solcher Serotonin-Rezeptoren wurde erstmals 1985 von Richardson et al. (Nature; Vol. 316; 126-131) postuliert und seitdem wurden zahlreiche 5-HT₃-Rezeptorantagonisten entwickelt wie z.B. ICS 205-930, MDL 72222, Odansetron, GR 65 630, Granisetron u.a.. Solche 5-HT₃-Rezeptorantagonisten zeigen unter anderem auch die für die Johanniskraut-Präparate bekannten Wirkungen gegen psychovegetative Störungen, depressive Verstimmungszustände, Angst und nervöse Unruhe etc.. Es liegt deshalb nahe, daß zusätzlich zu den bekannten Monoaminoxidase-hemmenden Wirkungen auch die antagonistische Wirkung der Johanniskrautinhaltsstoffe gegen 5-HT₃-Rezeptor-vermittelte Prozesse für die Wirksamkeit der Extrakte dieser Arzneipflanze verantwortlich ist.

Männliche Mäuse (NMRI) mit Körpergewicht zwischen 20 und 30 g dienten als Versuchstiere. Angewendet wurde eine von Saxena R.R. und Lawang A. (Arch. Int. Pharmacodyn.; 277; 235-252, 1985) beschriebene Methode. Bestimmt wurde der Einfluß eines bekannten HT₃-Rezeptorantagonisten (Cisapride) und der Extrakte A und B auf die durch Serotonin (0,25 mg/kg i.v.) induzierten Bradykardien in narkotisierten Tieren. Die Prüfsubstanzen wurden immer 30 min. vor der Serotoningabe i.p. appliziert.

Die erhaltenen Versuchsergebnisse sind in der folgenden Tabelle zusammengefaßt:

| Prüfsubstanz | Dosis | % Hemmung der Serotonininduzierten Bradykardien |
|---|---|---|
| Extrakt A (handelsüblicher Extrakt) | 500 | 61 |
| | 300 | 10 |
| Extrakt B (gemäß Beispiel 1) | 500 | 81 |
| | 100 | 70 |
| | 30 | 0 |
| Cisapride (Vergleich) | 5 | 80 |

Die Untersuchungsergebnisse zeigen, daß der erfindungsgemäß hergestellte Extrakt nicht nur hypericinarm ist und 5-HT₃-antagonistische Wirkungen besitzt, sondern auch in den Aktivkomponenten der Arzneipflanze (z.B. Hyperforin) angereichert ist, die für die 5-HT₃-rezeptorantagonistische Wirkung verantwortlich sind. Obwohl der Vergleichsextrakt A mehr als die 10fache Menge an Hypericin enthält, ist seine Wirkungsstärke mindestens 5mal schwächer als diejenige des erfindungsgemäß hergestellten Extrakts B.

Die Befunde, daß die Johanniskrautinhaltsstoffe die 5-HT₃-Rezeptorfunktionen antagonisieren, eröffnen eine Reihe von neuen therapeutischen Anwendungen der Zubereitungen dieser Arzneipflanze für die Therapie verschiedener Erkrankungen, die nicht unbedingt mit depressiven Verstimmungszuständen etc. einhergehen. Solche Erkrankungen sind u.a. Migräne, Erbrechen und andere gastrointestinale Störungen.

## Patentansprüche

1. Trockenextrakt aus dem Kraut von Hypericum perforatum L. (Johanniskraut), gekennzeichnet durch einen gegenüber dem Gesamt-Hypericingehalt der als Ausgangsmaterial eingesetzten Droge verminderten Gesamt-Hypericingehalt von weniger als 0,1 Gew.-% und durch einen gegenüber dem Gesamt-Hyperforingehalt der als Ausgangsmaterial eingesetzten Droge erhöhten Gesamt-Hyperforingehalt von mindestens 5 Gew.-%.

2. Trockenextrakt nach Anspruch 1, gekennzeichnet durch einen Gesamt-Hypericingehalt von weniger als 0,03 Gew.-%.

3. Trockenextrakt nach Anspruch 2, dadurch gekennzeichnet, daß er hypericinfrei ist.

4. Trockenextrakt nach einem der Ansprüche 1 bis 3, gekennzeichnet durch einen Gesamt-Hyperforingehalt von mindestens 10 Gew.-%.

5. Psychovegetativ und antidepressiv wirksames Arzneimittel, enthaltend einen Trockenextrakt gemäß einem der Ansprüche 1 bis 4, gegebenenfalls zusammen mit üblichen, pharmakologisch verträglichen Hilfs- und Zusatzstoffen.

6. Verwendung des Trockenextrakts gemäß einem der Ansprüche 1 bis 4 zur Herstellung psychovegetativ und antidepressiv wirksamer Arzneimittel.

7. Verwendung des Trockenextrakts gemäß einem der Ansprüche 1 bis 4, gegebenenfalls zusammen mit üblichen, pharmakologisch verträglichen Hilfs- und Zusatzstoffen, zur Herstellung eines Arzneimittels mit Serotonin-antagonistischer Wirksamkeit.

8. Verfahren zur Herstellung eines Trockenextrakts aus dem Kraut von Hypericum perforatum L., bei dem eine frische oder schonend getrocknete Johanniskrautdroge mit wäßrigem Ethanol extrahiert wird und aus dem so erhaltenen Flüssigextrakt in an sich bekannter Weise ein Trockenextrakt gewonnen wird, dadurch gekennzeichnet, daß dem Flüssigextrakt Polyvinylpyrrolidon oder Aktivkohle zugesetzt und gerührt wird, anschließend filtriert wird und die erhaltene Lösung zur Trockne eingeengt und gegebenenfalls nachgetrocknet wird.

9. Verfahren nach Anspruch 8, dadurch gekennzeichnet, daß dem Flüssigextrakt 30 bis 200 % Polyvinylpyrrolidon, bezogen auf die Masse des Trockenextrakts, zugesetzt werden.

10. Verfahren nach Anspruch 8, dadurch gekennzeichnet, daß dem Flüssigextrakt 5 bis 50 % Aktivkohle, bezogen auf die Masse des Trockenextrakts, zugesetzt werden.

11. Verfahren zur Herstellung eines Trockenextrakts aus dem Kraut von Hypericum perforatum L., bei dem eine frische oder schonend getrocknete Johanniskraut-Droge mit wäßrigem Ethanol extrahiert und der erhaltene Flüssigextrakt (Primärextrakt) durch Abziehen des Lösungsmittels unter vermindertem Druck zur Trockne eingeengt wird, dadurch gekennzeichnet, daß der Primärextrakt in einem Dioxan-Wasser-Gemisch gelöst und die so erhaltene Lösung säulenchromatographisch unter Verwendung von Dioxan/Wasser als Eluens in zwei Fraktionen aufgetrennt wird, wonach die zuerst aufgefangene Fraktion, die einen verminderten Gesamt-Hypericingehalt aufweist, zur Trockne eingeengt und gegebenenfalls nachgetrocknet wird.

12. Verfahren nach Anspruch 11, dadurch gekennzeichnet, daß der Primärextrakt in einem Gemisch aus 7 Volumteilen Dioxan und 3 Volumteilen Wasser gelöst wird.

13. Verfahren nach Anspruch 11 oder 12, dadurch gekennzeichnet, daß als Eluens für die säulenchromatographische Trennung ein Gemisch aus 7 Volumteilen Dioxan und 3 Volumteilen Wasser verwendet wird.

14. Verfahren nach einem der Ansprüche 11 bis 13, dadurch gekennzeichnet, daß die säulenchromatographische Trennung als Gelchromatographie durchgeführt wird.

15. Verfahren zur Herstellung eines Trockenextrakts aus dem Kraut von Hypericum perforatum L., bei dem eine frische oder schonend getrocknete Johanniskraut-Droge mit wäßrigem Ethanol extrahiert wird, dadurch gekennzeichnet, daß der so erhaltene Flüssigextrakt (Primärextrakt) zwischen n-Butanol und Wasser verteilt wird, sodann die n-Butanol-Phase zur Trockne eingeengt und anschließend gelchromatographisch fraktioniert wird.

16. Verfahren nach einem der Ansprüche 8 bis 15, dadurch gekennzeichnet, daß die Droge mit 50 bis 96 Gew.-%igem Ethanol extrahiert wird.

## Claims

1. A dry extract from the leaves of hypericum perforatum L. (St. John's wort), characterized by a total content of hypericin of less than 0.1% by weight, being reduced as compared with the total content of hypericin of the drug used as a starting material, and a total content of hyperforin of at least 5% by weight, being increased as compared with the total content of hyperforin of the drug used as the starting material.

2. The dry extract of claim 1, characterized by a total content of hypericin of less than 0.03% by weight.

3. The dry extract of claim 2, characterized by being free of hypericin.

4. The dry extract of any one of claims 1 to 3, characterized by a total content of hyperforin of at least 10% by weight.

5. A pharmaceutical composition being psychovegetatively and antidepressively effective containing a dry extract according to any one of claims 1 to 4, optionally together with conventional, pharmacologically acceptable auxiliary agents and additives.

6. Use of the dry extract of any one of claims 1 to 4 for the manufacture of psychovegetatively and antidepressively effective pharmaceutical compositions.

7. Use of the dry extract of any one of claims 1 to 4, optionally together with conventional, pharmacologically acceptable auxiliary agents and additives, for the manufacture of a pharmaceutical composition having serotonin-antagonistic activity.

8. A process for the manufacture of a dry extract from the leaves of hypericum perforatum L. comprising extracting a fresh or mildly dried drug of St. John's wort with aqueous ethanol and recovering from the thus obtained liquid extract a dry extract in a per se known manner, characterized in that polyvinylpyrrolidone or activated charcoal is added to and stirred with said liquid extract, subsequently filtered, and the resulting solution is evaporated to dryness and, optionally, post-dried.

9. The process of claim 8, characterized in that 30 to 200% polyvinylpyrrolidone, based upon the mass of said dry extract, are added to said liquid extract.

10. The process of claim 8, characterized in that 5 to 50% activated charcoal, based upon the mass of said dry extract, are added to said liquid extract.

11. A process for the manufacture of a dry extract from the leaves of hypericum perforatum L. comprising extracting a fresh or mildly dried drug of St. John's wort with aqueous ethanol and evaporating the obtained liquid extract (primary extract) to dryness by means of evaporating the solvent under reduced pressure, characterized in that said primary extract is dissolved in a mixture of dioxane and water and that the thus obtained solution is separated into two fractions by column chromatography using dioxane/water as an eluent, that thereafter the fraction first recovered which has a reduced total content of hypericin, is evaporated to dryness and, optionally, post-dried.

12. The process of claim 11, characterized in that said primary extract is dissolved in a mixture of 7 parts by volume of dioxane and 3 parts by volume of water.

13. The process of claim 11 or 12, characterized in that a mixture of 7 parts by volume of dioxane and 3 parts by volume of water is used as said eluent for the separation by means of column chromatography.

14. The process of any one of claims 11 to 13, characterized in that said separation by means of column chromatography is conducted as a gel chromatography.

15. A process for the manufacture of a dry extract from the leaves of hypericum perforatum L. comprising extracting a fresh or mildly dried drug of St. John's wort with aqueous ethanol, characterized in that the thus obtained liquid extract (primary extract) is partitioned between n-butanol and water, said n-butanol phase is then evaporated to dryness and, subsequently, is fractioned by means of gel chromatography.

16. The process of any one of claims 8 to 15, characterized in that said drug is extracted with ethanol having a concentration of 50 to 96% by weight.

## Revendications

1. Extrait sec de la plante herbacée de Hypericum perforatum L. (millepertuis), caractérisé par une teneur en hypericine totale, abaissée par rapport à la teneur en hypericine totale de la drogue utilisée comme matière première, de moins de 0,1 % en poids et par une teneur en hyperforine totale, accrue par rapport à la teneur en hyperforine totale de la drogue utilisée comme matière première, d'au moins 5 % en poids.

2. Extrait sec selon la revendication 1, caractérisé par une teneur en hypericine totale de moins de 0,03 % en poids.

3. Extrait sec selon la revendication 2, caractérisé par le fait qu'il est exempt d'hypericine.

4. Extrait sec selon l'une des revendications 1 à 3, caractérisé par une teneur en hyperforine totale d'au moins 10 % en poids.

5. Médicament à activité psychovégétative et anti-dépressive, contenant un extrait sec selon l'une des revendications 1 à 4, éventuellement conjointement avec des adjuvants et additifs pharmacologiquement acceptables classiques.

6. Utilisation de l'extrait sec selon l'une des revendications 1 à 4 pour la préparation d'un médicament à activité psychovégétative et anti-dépressive.

7. Utilisation de l'extrait sec selon l'une des revendications 1 à 4, éventuellement conjointement avec des adjuvants et des additifs pharmacologiquement acceptables classiques, pour la préparation d'un médicament ayant une activité d'antagoniste de la sérotonine.

8. Procédé pour la préparation d'un extrait sec de la plante herbacée de Hypericum perforatum L., dans lequel une drogue de millepertuis fraîche ou séchée avec ménagement est extraite avec de l'éthanol aqueux et un extrait sec est obtenu de manière connue en soi à partir de l'extrait liquide ainsi obtenu, caractérisé par le fait qu'on ajoute, à l'extrait liquide, de la polyvinylpyrrolidone ou du charbon actif et on agite, ensuite on filtre et on concentre jusqu'à siccité la solution obtenue et on procède éventuellement à un séchage final.

9. Procédé selon la revendication 8, caractérisé par le fait qu'on ajoute à l'extrait liquide 30 à 200 % de polyvinylpyrrolidone, par rapport à la masse de l'extrait sec.

10. Procédé selon la revendication 8, caractérisé par le fait qu'on ajoute à l'extrait liquide 5 à 50 % de charbon actif, par rapport à la masse de l'extrait sec.

11. Procédé pour la préparation d'un extrait sec de la plante herbacée de Hypericum perforatum L., dans lequel une drogue de millepertuis fraîche ou séchée avec ménagement est extraite avec de l'éthanol aqueux et l'extrait liquide obtenu (extrait primaire) est concentré jusqu'à siccité par élimination du solvant sous pression réduite, caractérisé par le fait que l'extrait primaire est dissous dans un mélange dioxanne-eau, et la solution ainsi obtenue est séparée en deux fractions par chromatographie sur colonne avec utilisation de dioxanne/eau comme éluant, après quoi la fraction recueillie en premier, qui présente une teneur en hypericine totale abaissée, est concentrée jusqu'à siccité et éventuellement soumise à un séchage final.

12. Procédé selon la revendication 11, caractérisé par le fait que l'extrait primaire est dissous dans un mélange de 7 parties en volume de dioxanne et 3 parties en volume d'eau.

13. Procédé selon la revendication 11 ou 12, caractérisé par le fait qu'on utilise comme éluant, pour la séparation par chromatographie sur colonne, un mélange de 7 parties en volume de dioxanne et 3 parties en volume d'eau.

14. Procédé selon l'une des revendications 11 à 13, caractérisé par le fait qu'on effectue la séparation par chromatographie sur colonne sous forme de chromatographie sur gel.

15. Procédé pour la préparation d'un extrait sec de la plante herbacée de Hypericum perforatum L., dans lequel une drogue de millepertuis fraîche ou séchée avec ménagement est extraite avec de l'éthanol aqueux, caractérisé par le fait que l'extrait liquide ainsi obtenu (extrait primaire) est réparti entre du n-butanol et de l'eau, après quoi la phase de n-butanol est concentrée jusqu'à siccité et ensuite fractionnée par chromatographie sur gel.

16. Procédé selon l'une des revendications 8 à 15, caractérisé par le fait que la drogue est extraite avec de l'éthanol à 50 à 96 % en poids.
